# EUROPEAN PATENT SPECIFICATION

(11) **EP 2 242 474 B1**
(45) Date of publication and mention of the grant of the patent: **30.11.2011**
(21) Application number: 09713501.6
(22) Date of filing: 29.01.2009
(51) Int. Cl.: A61K 8/73, A61K 8/81, A61Q 1/12, A61Q 19/00

(54) **CATIONIC COPOLYMER AND STARCHES FORMULATED COSMETIC COMPOSITIONS EXHIBITING RADIANCE WITH SOFT FOCUS**
KOSMETISCHE ZUSAMMENSETZUNGEN ENTHALTEND EIN KATIONISCHES COPOLYMER UND STÄRKE FÜR EIN STRAHLENDES HAUTBILD MIT WEICHEM FOKUS
COMPOSITIONS COSMÉTIQUES CONTENANT UN COPOLYMÈRE CATIONIQUE ET UN AMIDON PRÉSENTANT UNE BRILLANCE À EFFET DE FLOU

(30) Priority: 19.02.2008 US 29657
(43) Date of publication of application: 27.10.2010
(73) Proprietor: Unilever PLC, London EC4Y 0DY (GB); Unilever N.V., 3013 AL Rotterdam (NL)
(72) Inventor: POLONKA, Jack, Trumbull, Connecticut 06611 (US); BARTOLONE, John, Brian, Trumbull, Connecticut 06611 (US)
(74) Representative: Acham, Nicholas Clive
(86) International application number: PCT/EP2009/051031
(87) International publication number: WO 2009/103603

(56) References cited:
- EP-A- 1 557 154
- WO-A-94/02176
- US-A1- 2005 014 893
- US-A1- 2005 220 736
- US-A1- 2005 255 058
- US-A1- 2007 237 730

## Description

The invention relates to compositions for improving the appearance of skin, particularly to provide good coverage over imperfections such as pores and uneven skin tone, while retaining a natural skin appearance.

A matte effect is desired for users of color cosmetics. The matte finish overcomes the shiny effect engendered by greasy skin, particularly under hot and humid conditions. Absorbent fillers such as talc, silica, kaolin and other inorganic particulates have been used to achieve the effect by their optical properties.

Imperfect skin can be hidden in two ways through manipulation of light transmission. In the first, components of the color cosmetic may simply reflect light back toward the source. An alternative approach is referred to as achieving a soft focus effect. Here the incoming light is distorted by scattering (lensing). Components of the color cosmetic in this mechanism operate as lenses to bend and twist light into a variety of directions.

While it is desirable to hide imperfect skin through a matte effect, there is also a desire to achieve a healthy skin radiance. A cosmetic covering that is too opaque hides the skin under a paint-like coating. Imperfections are hidden but there is no radiance. Where light transmission is insufficiently hindered, the opposite occurs. Here the glow may be healthy but aesthetically displeasing skin topography and color may now be apparent.

US 5 997 890 (Sine et al.) US Patent 5 972 359 (Sine et al.) and US 6 174 533 B1 (SaNogueira, Jr.) are all directed to topical compositions to provide good coverage of skin imperfections. The solution proposed by these documents is the use of a metal oxide with a refractive index of at least 2 and a neat primary particle size of from 100 to 300 nm. Preferred particulates are titanium dioxide, zirconium oxide and zinc oxide.

Silicone gelling agents such as crosslinked organopolysiloxane elastomers because of their excellent skinfeel properties have been found useful in make-up compositions. For instance, US 5 266 321 (Shukuzaki et al.) discloses an oily make-up composition comprised of a silicone gel crosslinked elastomer, titanium dioxide, mica and iron oxides. Japanese patent application 61-194009 (Harashima) describes a make-up composition comprising a cured organopolysiloxane elastomer powder and pigments which may be selected from talc, titanium dioxide, zinc oxide and iron oxides.

WO 94/02176 A1 describes cosmetic compositions to improve the appearance of the skin containing a starch derivative and a cationic polymer having (methacryloyl)oxyethyl trimethylammonium units.

A challenge which has not been fully met by the known art is delivery of a composition with appropriate optics to achieve both soft focus and radiance properties in a system that still provides excellent skinfeel.

### SUMMARY OF THE INVENTION

A cosmetic composition is provided which includes:
(i) from 0.1 to 30% by weight of a starch;
(ii) from 0.1 to 20% by weight of a cationic copolymer having monomer units of acryloylethyl tri(C₁-C₃ alkyl) ammonium salt; and
(iii) a cosmetically acceptable carrier, wherein the cationic copolymer is acrylamide / acryloylethyl trimethylammonium cholride / tris(hydroxymethyl)-acrylamidomethane copolymer.

### DETAILED DESCRIPTION OF THE INVEIVTION

Now it has been observed that a soft focus effect with radiance can be obtained by a combination of starch and a cationic copolymer wherein one of the monomer units is acryloylethyl tri(C₁-C₃ alkyl) ammonium salt, as defined above.

### Starch

The term "starch" or "starches" for purposes of this invention mean not only native but also physically and chemically modified starches. Physically modified starches include gelatinized starches, fully or partially hydrated starches and destructurized starches as well as crosslinked starches. Chemically modified varieties are those that have undergone acylation, alkylation, epoxidization, quaternization, carboxylation, phosphorylation, etherification (eg reaction with propylene or ethylene oxide), esterification (eg reaction with acetic anhydride) to mention but a few for illustrative purposes. Particularly preferred chemically modified varieties are aluminum starch octenyl succinate (Dry Flo® available from the National Starch and Chemical Company), and sodium hydroxypropyl starch phosphate (Pure Gel B994 available from Grain Processing Corporation). Typical starches may be selected from tapioca, corn, barley, spelt, potato, sweet potato, banana, wheat, rice, sago, amaranth, sorghum, waxy maize, waxy tapioca, waxy potato, and high amylase starches containing greater than 40% amylase and the like. Particularly preferred starches are tapioca and sodium hydroxypropyl starch phosphate.

Amounts of the starch range from 0.1 to 30%, preferably from 1 to 15%, optimally from 3 to 8% by weight of the composition.

### Cationic copolymer

The cationic copolymer of the present invention incorporates as one of the repeating units an acryloylethyl tri(C₁-C₃ alkyl) ammonium salt. The term "salt" for this monomer unit may be but is not limited to chloride, bromide, sulfate, sulphonate, methosulfate, nitrate, tosylate, phosphate and phosphonate. The term "copolymer" means at least two different monomer repeating units, preferably three or more different monomer repeating units. Monomer units that crosslink are particularly useful.

The cationic copolymer is acrylamide/acryloylethyl trimethylammonium chloride/tris(hydroxymethyl)-acrylamidomethane copolymer, commercially available under the trademark 7688 MP from Seppic Inc.

Number average molecular weight of the copolymer according to the invention may range from 1,000 to 3,000,000, preferably from 3,000 to 100,000, optimally from 10,000 to 80,000.

Amounts of the copolymer range from 0.1 to 20%, preferably from 0.5 to 10%, more preferably from 1 to 7%, and optimally from 1.5 to 5% by weight of the composition.

### Cosmetically acceptable carrier

Compositions of this invention will also include a cosmetically acceptable carrier. Amounts of the carrier may range from 1 to 99.9%, preferably from 70 to 95%, optimally from 80 to 90% by weight of the composition. Among the useful carriers are water, emollients, fatty acids, fatty alcohols, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from 5 to 95%, preferably from 20 to 70%, optimally from 35 to 60% by weight of the composition.

Emollient materials may serve as cosmetically acceptable carriers. These may be in the form of silicone oils, natural or synthetic esters and hydrocarbons. Amounts of the emollients may range anywhere from 0.1 to 95%, preferably between 1 and 50% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from 5 x 10⁻⁶ to 0.1 m²/s at 25°C. Among the preferred non-volatile emollients useful in the present compositions are the polydimethyl siloxanes having viscosities from 1 x 10⁻⁵ to 4 x 10⁻⁴ m²/s at 25°C.

Another class of non-volatile silicones are emulsifying and non-emulsifying silicone elastomers. Representative of this category is dimethicone/vinyl dimethicone crosspolymer available as Dow Corning 9040, General Electric SFE 839, and Shin-Etsu KSG-18. Silicone waxes such as Silwax WS-L (dimethicone copolyol laurate) may also be useful.

Among the ester emollients are:
a) Alkyl esters of saturated fatty acids having 10 to 24 carbon atoms. Examples thereof include behenyl neopentanoate, isononyl isonanonoate, isopropyl myristate and octyl stearate.
b) Ether-esters such as fatty acid esters of ethoxylated saturated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from 0.1 to 20% by weight of the compositions.

Hydrocarbons which are suitable cosmetically acceptable carriers include petrolatum, mineral oil, C₁₁-C₁₃ isoparaffins, polybutenes, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol.

Thickeners can be utilized as part of the cosmetically acceptable carrier of compositions according to the present invention. Typical thickeners include crosslinked acrylates (eg Carbopol 982®), hydrophobically-modified acrylates (eg Carbopol 1382®), polyacrylamides (eg Sepigel 305®), acryloylmethylpropane sulfonic acid/salt polymers and copolymers (eg Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, calcium carbonate and silicates such as magnesium aluminum silicate (Veegum®). Amounts of the thickener may range from 0.0001 to 10%, usually from 0.001 to 1%, optimally from 0.01 to 0.5% by weight of the composition.

Humectants may be employed in the present invention. These are generally polyhydric alcohol-type materials. Typical polyhydric alcohols include glycerol, propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of adjunct humectant may range anywhere from 0.5 to 50%, preferably between 1 and 15% by weight of the composition.

Compositions of the present invention may be in any form. These forms may include lotions, creams, roll-on formulations, sticks, mousses, aerosol and non-aerosol sprays and fabric (eg non-woven textile) applied formulations.

Surfactants may also be present in compositions of the present invention. Total concentration of the surfactant when present may range from 0.1 to 30%, preferably from 0.1 to 15%, optimally from 0.5 to 2% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (eg methyl gluconamides) and trialkylamine oxides are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof.

Useful amphoteric surfactants include cocoamidopropyl betaine, C₁₂-C₂₀ trialkyl betaines, sodium lauroamphoacetate and sodium laurodiamphoacetate.

Sunscreen actives may also be included in compositions of the present invention. These will be organic compounds having at least one chromophoric group absorbing within the ultraviolet ranging from 290 to 400 nm. Chromophoric organic sunscreen agents may be divided into the following categories (with specific examples) including: p-aminobenzoic acid, its salts and its derivatives (ethyl, isobutyl, glyceryl esters; p-dimethylaminobenzoic acid); anthranilates (o-aminobenzoates; methyl, menthyl, phenyl, benzyl, phenylethyl, linalyl, terpinyl, and cyclohexenyl esters); salicylates (octyl, amyl, phenyl, benzyl, menthyl, glyceryl, and dipropyleneglycol esters); cinnamic acid derivatives (menthyl and benzyl esters, alpha-phenyl cinnamonitrile; butyl cinnamoyl pyruvate); dihydroxycinnamic acid derivatives (umbelliferone, methylumbelliferone, methylaceto-umbelliferone); trihydroxycinnamic acid derivatives (esculetin, methylesculetin, daphnetin, and the glucosides, esculin and daphnin); hydrocarbons (diphenylbutadiene, stilbene); Dibenzalacetone and benzalacetophenone; naphtholsulfonates (sodium salts of 2-naphthol-3,6-disulfonic and of 2-naphthol-6,8-disulfonic acids); dihydroxy-naphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); Hydroxy- or methoxy-substituted benzophenones; uric and vilouric acids; tannic acid and its derivatives (eg, hexaethylether); (butyl carbityl) (6-propyl piperonyl) ether; hydroquinone; benzophenon e s (oxybenzone, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxydibenzoylmethane; etocrylene; and 4-isopropyl-dibenzoylmethane). Particularly useful are: 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl 4-[bis(hydroxypropyl)]a minobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid and mixtures thereof.

Particularly preferred are such materials as ethylhexyl p-methoxycinnamate (available as Parsol MCX®), Avobenzene (available as Parsol 1789®) and Dermablock OS® (octylsalicylate).

Amounts of the organic sunscreen agent will range from 0.1 to 15%, preferably from 0.5% to 10%, optimally from 1% to 8% by weight of the composition.

Advantageously present may also be water-insoluble organic material in the form of polymeric porous spherical particles. By the term "porous" is meant an open or closed cell structure. Preferably the particles are not hollow beads. Volume average particle size may range from 0.1 to 100, preferably from 1 to 50, more preferably greater than 5 and especially from 5 to 15, optimally from 6 to 10 µm. Organic polymers or copolymers are the preferred materials and can be formed from monomers including the acid, salt or ester forms of acrylic acid and methacrylic acid, methylacrylate, ethylacrylate, ethylene, propylene, vinylidene chloride, acrylonitrile, maleic acid, vinyl pyrrolidone, styrene, butadiene and mixtures thereof. The polymers are especially useful in cross-linked form. Cells of the porous particles may be filled by a gas which can be air, nitrogen or a hydrocarbon. Oil Absorbance (castor oil) is a measure of porosity and in the preferred but not limiting embodiment may range from 90 to 500, preferably from 100 to 200, optimally from 120 to 180 ml/100 grams. Density of the particles in the preferred but not limiting embodiment may range from 0.08 to 0.55, preferably from 0.15 to 0.48 g/cm³.

Illustrative porous polymers include polymethylmethacrylate and cross-linked polystyrene. Most preferred is polymethyl methacrylate (available as Ganzpearl® GMP 820 from Presperse, Inc., Piscataway, New Jersey).

Amounts of the water-insoluble polymeric porous particles may range from 0.01 to 10%, preferably from 0.1 to 5%, optimally from 0.3 to 2% by weight of the composition.

Preservatives can desirably be incorporated into the compositions of this invention to protect against the growth of potentially harmful microorganisms. Particularly preferred preservatives are phenoxyethanol, methyl paraben, propyl paraben, imidazolidinyl urea, dimethyloldimethylhydantoin, ethylenediaminetetraacetic acid salts (EDTA), sodium dehydroacetate, methylchloroisothiazolinone, methylisothiazolinone, iodopropynbutylcarbamate and benzyl alcohol. The preservatives should be selected having regard for the use of the composition and possible incompatibilities between the preservatives and other ingredients. Preservatives are preferably employed in amounts ranging from 0.01% to 2% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from green tea, chamomile, licorice, aloe vera, grape seed, citrus unshui, willow bark, sage, thyme and rosemary.

Also included may be such materials as lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Amounts of these materials may range from 0.000001 to 10%, preferably from 0.0001 to 1% by weight of the composition.

Colorants, opacifiers and abrasives may also be included in compositions of the present invention. Each of these substances may range from 0.05 to 5%, preferably between 0.1 and 3% by weight of the composition.

Still other suitable actives for skin compositions and use in the present invention include creatine, resveratrol, hyaluronic acid (particularly those of molecular weight of around 800), and combinations thereof. Amounts may range from 0.000001 to 5%, preferably from 0.001 to 1% by weight of the compositions.

Compositions of the present invention may also contain vitamins. Illustrative watersoluble vitamins are niacinamide, vitamin B₂, vitamin B₆, vitamin C and biotin. Among the useful water-insoluble vitamins are vitamin A (retinol), vitamin A palmitate, ascorbyl tetraisopalmitate, vitamin E (tocopherol), vitamin E Acetate and DL-panthenol. Total amount of vitamins when present in compositions according to the present invention may range from 0.001 to 10%, preferably from 0.01% to 1%, optimally from 0.1 to 0.5% by weight of the composition.

Desquamation agents are further optional components. Illustrative are the alpha-hydroxycarboxylic acids and beta-hydroxycarboxylic acids and salts of these acids. Among the former are salts of glycolic acid, lactic acid and malic acid. Salicylic acid is representative of the beta-hydroxycarboxylic acids. Amounts of these materials when present may range from 0.1 to 15% by weight of the composition.

Except in the operating and comparative examples, or where otherwise explicitly indicated, all numbers in this description indicating amounts of material ought to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

All documents referred to herein, including all patents, patent applications, and printed publications, are hereby incorporated by reference in their entirety in this disclosure.

The following examples will more fully illustrate the embodiments of this invention. All parts, percentages and proportions referred to herein and in the appended claims are by weight unless otherwise illustrated.

### EXAMPLES 1 - 8

Formulas suitable for the present invention are recorded in table I.

### EXAMPLES 9-17

A series of experiments were conducted to demonstrate the radiance and soft focus effects of compositions according to the present invention. Table II outlines the composition of the formulas and performance results.

Radiance in the form of a gloss measurement was evaluated on a Novogloss® Glossmeter. The Glossmeter geometry was first set with both detector and light source at 85° from normal. An appropriate reflection standard was used to calibrate the instrument. Gloss (radiance) is reported as the percent difference in before and after treatment measurements. The larger the value (or less negative), the better the radiance effect.

A haze determination was utilized to evaluate soft focus effects. For this purpose, a Hunter Lab Spectracolorimeter was employed. This instrument had an optical geometry of 0° incidence and 45° reflectance (both for normal). Reflectance measurements gauge the soft focus effect from an opaque surface. These measurements are reported as a Haze value. It is the difference between an initial (zero) reading and a final one after treatment. Higher Haze values indicate a greater soft focus effect.

Sample formula in 20 mg dosage was applied onto a human forearm, and let dry for 20 minutes. Treated forearms were then rinsed under water for 2 minutes, and let dry for another 20 minutes. Thereafter the treated areas were scanned on the Hunter Lab Spectracolorimeter and also on the Glossmeter. Before and after changes were recorded both for pre-rinse and post-rinse conditions to obtain the respective percent Gloss and Haze values.

Example 9 is a control. No significant change over untreated surface is seen in radiance/Gloss or soft focus/Haze. Example 10 contained a starch component (Pure Gel B894® which is sodium hydroxypropyl starch phosphate) but no cationic copolymer. This formula exhibited good properties in dry state but upon rinsing lost both radiance and soft focus (-3.4 and 4.1, respectively). Moreover, this formula was sticky/tacky in feel.

Examples 11, 12 and 13 were each formulated with a cationic copolymer but no starch. Merquat 5® is a trademark for acrylamide/methacryloyloxyethyl trimethyl ammonium methylsulfate copolymer; Simulgel INS® is a trademark for hydroxyethylacrylate/sodium acryloyldimethyltaurate copolymer; and copolymer 7688 MP is a trademark for acrylamide/acryloylethyl trimethylammonium chloride/tris(hydroxymethyl)acrylamidomethane copolymer. Examples 11, 12 and 13 exhibited an increased radiance but there was hardly any change in soft focus/haze.

Examples 14 - 17 included both a cationic copolymer and a starch. Example 14 with Simulgel INS® as the copolymer exhibited the most loss in Haze (soft focus) after rinse. Merquat 5® in example 16 after rinse retained some residual Haze effect. Best performance was with examples 15 and 17 containing the inventive copolymer 7688 MP. These formulas maintained not only a good soft focus (Haze) effect under dry conditions but also experienced little loss of this property on rinsing.

Based on the foregoing experiments, it is seen possible to improve radiance (Gloss) while maintaining soft focus (Haze) through use of starch in combination with an acryloylethyl trialkyl ammonium salt copolymer such as 7688 MP. Simulgel INS® had similar properties to 7688 MP except the former completely lacked substantivity against rinse-off. Merquat 5® suffered from poor film formation characteristics which hindered gloss enhancement as well as having lower substantivity relative to 7688 MP. From a sensory perspective, 7688 MP in combination with starch revealed a silky feel. By contrast, Merquat 5® and starch formulas felt tacky/sticky.

**TABLE I**

| Component | Formula (Weight %) | | | | | | | |
|---|---|---|---|---|---|---|---|---|
| | 1 | 2 | 3 | 4 | 5 | 6 | 7 | 8 |
| Cyclopentasiloxane | 20.00 | 20.00 | 0.00 | 0.00 | 0.00 | 10.00 | 10.00 | 10.00 |
| Cationic copolymer (7688 MP) | 10.00 | 0.50 | 20.00 | 20.00 | 8.00 | 1.00 | 2.00 | 0.50 |
| Ethylhexyl methoxycinnamate | 4.00 | 4.00 | 4.00 | 4.00 | 4.00 | 4.00 | 4.00 | 4.00 |
| Ethylhexyl salicylate | 2.00 | 2.00 | 2.00 | 2.00 | 2.00 | 2.00 | 2.00 | 2.00 |
| Polysorbate 40 | 1.62 | 1.62 | 1.62 | 1.62 | 1.62 | 1.62 | 1.62 | 1.62 |
| Cetyl alcohol | 1.55 | 1.55 | 1.55 | 1.55 | 1.55 | 1.55 | 1.55 | 1.55 |
| Dimethicone | 1.00 | 1.00 | 1.00 | 1.00 | 1.00 | 1.00 | 1.00 | 1.00 |
| Titanium dioxide coated mica (Timiron® MP111) | 1.00 | 0.80 | 0.80 | 0.80 | 1.20 | 1.20 | 1.00 | 0.80 |
| Polymethylmethacrylate beads (Ganzpearl® GMP 0820) | 0.80 | 0.80 | 0.80 | 0.80 | 0.80 | 0.40 | 0.60 | 1.00 |
| Glycerin Monostearate | 0.78 | 0.78 | 0.78 | 0.78 | 0.78 | 0.78 | 0.78 | 0.78 |
| Sodium hydroxypropyl starch phosphate | 1.50 | 2.50 | 1.50 | 0.50 | 0.00 | 0.00 | 0.00 | 0.00 |
| Tapioca starch | 0.00 | 0.00 | 0.00 | 0.00 | 2.50 | 0.50 | 1.50 | 1.00 |
| Stearic acid | 0.25 | 0.25 | 0.25 | 0.25 | 0.25 | 0.25 | 0.25 | 0.25 |
| Cholesterol | 0.20 | 0.20 | 0.20 | 0.20 | 0.20 | 0.20 | 0.20 | 0.20 |
| Linoleic acid | 0.10 | 0.10 | 0.10 | 0.10 | 0.10 | 0.10 | 0.10 | 0.10 |
| Water | Balance | Balance | Balance | Balance | Balance | Balance | Balance | Balance |

**TABLE II**

| | Example (Weight %) | | | | | | | | |
|---|---|---|---|---|---|---|---|---|---|
| Component | 9 | 10 | 11 | 12 | 13 | 14 | 15 | 16 | 17 |
| Glyceryl monostearate | 2.4 | 2.4 | 2.4 | 2.4 | 2.4 | 2.4 | 2.4 | 2.4 | 2.4 |
| Cetyl alcohol | 2.4 | 2.4 | 2.4 | 2.4 | 2.4 | 2.4 | 2.4 | 2.4 | 2.4 |
| PEG-100 stearate | 1.2 | 1.2 | 1.2 | 1.2 | 1.2 | 1.2 | 1.2 | 1.2 | 1.2 |
| Glycerin | 13 | 13 | 13 | 13 | 13 | 13 | 13 | 13 | 13 |
| Merquat 5® | -- | -- | -- | -- | 1.0 | -- | -- | 1.0 | -- |
| Cationic copolymer (7688MP) | -- | -- | -- | 1.0 | -- | -- | 1.0 | -- | 1.0 |
| Simulgel INS® | -- | -- | 1.0 | -- | -- | 1.0 | - | -- | -- |
| Pure Gel B994® | -- | 2.0 | -- | -- | -- | 2.0 | 2.0 | 2.0 | -- |
| Tapioca | -- | -- | -- | -- | -- | -- | -- | -- | 2.0 |
| Ethylhexyl methoxycinnamate | 1.25 | 1.25 | 1.25 | 1.25 | 1.25 | 1.25 | 1.25 | 1.25 | 1.25 |
| Dimethicone 50 cst | 3 | 3 | 3 | 3 | 3 | 3 | 3 | 3 | 3 |
| Titanium dioxide | 0.1 | 0.1 | 0.1 | 0.1 | 0.1 | 0.1 | 0.1 | 0.1 | 0.1 |
| Lactic acid and potassium lactate | 9.85 | 9.85 | 9.85 | 9.85 | 9.85 | 9.85 | 9.85 | 9.85 | 9.85 |
| Olea Europaea(Olive) fruit oil | 1 | 1 | 1 | 1 | 1 | 1 | 1 | 1 | 1 |
| Methylparaben | 0.2 | 0.2 | 0.2 | 0.2 | 0.2 | 0.2 | 0.2 | 0.2 | 0.2 |
| Disodium EDTA | 0.05 | 0.05 | 0.05 | 0.05 | 0.05 | 0.05 | 0.05 | 0.05 | 0.05 |
| Propylparaben | 0.1 | 0.1 | 0.1 | 0.1 | 0.1 | 0.1 | 0.1 | 0.1 | 0.1 |
| Phenoxyethanol | 0.4 | 0.4 | 0.4 | 0.4 | 0.4 | 0.4 | 0.4 | 0.4 | 0.4 |
| Deionized water | 65.05 | 63.05 | 64.05 | 64.05 | 64.05 | 62.05 | 62.05 | 62.05 | 62.05 |
| **Optical Measurement Values After Application** | | | | | | | | | |
| % Gloss Change* | 1 | -30.2 | 8.7 | 10.2 | 6.3 | -20.4 | -15.6 | -27.3 | -16.3 |
| Haze | 0.1 | 25.2 | 0.2 | 0.1 | 0.3 | 25.6 | 26.3 | 25.5 | 26.4 |
| **Optical Measurement Values After Rinsing the Application** | | | | | | | | | |
| % Gloss Change | 0.0 | -3.4 | 1.4 | 4.4 | 2.1 | -2.4 | -14.3 | -7.2 | -15.4 |
| Haze | 0.1 | 4.1 | 0.1 | 0.2 | 0.1 | 3.8 | 23.6 | 11.8 | 24.2 |

| | | | | | | | | | |
|---|---|---|---|---|---|---|---|---|---|
| * Initial Gloss value of untreated forearm was 11.2. | | | | | | | | | |

## Claims

1. A cosmetic composition comprising:
(i) from 0.1 to 30% by weight of a starch;
(ii) from 0.1 to 20% by weight of a cationic copolymer comprising monomer units of acryloylethyl tri(C₁-C₃ alkyl) ammonium salt; and
(iii) a cosmetically acceptable carrier,
wherein the cationic copolymer is acrylamide/acryloylethyl trimethylammonium chloride/tris(hydroxymethyl)-acrylamidomethane copolymer.

2. A composition according to claim 1 wherein the starch is selected from the group consisting of aluminum starch octenylsuccinate and sodium hydroxypropyl starch phosphate .

3. A composition according to claim 1 wherein the starch is tapioca starch.

## Patentansprüche

1. Kosmetische Zusammensetzung, umfassend:
(i) 0,1 bis 30 Gew.-% einer Stärke;
(ii) 0,1 bis 20 Gew.-% eines kationischen Copolymers, das Monomereinheiten aus Acryloylethyl-tri(C₁-C₃-alkyl)-ammoniumsalz umfasst, und
(iii) einen kosmetisch verträglichen Träger,
wobei das kationische Copolymer ein Acrylamid/Acryloylethyltrimethylammoniumchlorid/Tris(hydroxymethyl)-acrylamidomethan-Copolymer ist.

2. Zusammensetzung gemäß Anspruch 1, wobei die Stärke aus der Gruppe, bestehend aus Aluminium-stärke-octenylsuccinat und Natriumhydroxypropyl-stärke-phosphat, ausgewählt ist.

3. Zusammensetzung gemäß Anspruch 1, wobei die Stärke Tapiokastärke ist.

## Revendications

1. Composition cosmétique comprenant :
(i) de 0,1 à 30 % en poids d'un amidon ;
(ii) de 0,1 à 20 % en poids d'un copolymère cationique comprenant des motifs monomères de sel d'acryloyléthyltri(alkyl(C₁-C₃))ammonium ; et
(iii) un véhicule acceptable sur le plan cosmétique,
dans laquelle le copolymère cationique est un copolymère d'acrylamide/chlorure d'acryloyl-éthyltriméthylammonium/tris(hydroxyméthyl)acryl-amidométhane.

2. Composition selon la revendication 1, dans laquelle l'amidon est choisi dans le groupe constitué par l'octénylsuccinate d'aluminium et d'amidon et le phosphate de sodium d'amidon hydroxypropylé.

3. Composition selon la revendication 1, dans laquelle l'amidon est l'amidon de tapioca.
